# EUROPEAN PATENT APPLICATION

(11) **EP 4 715 380 A1**
(43) Date of publication of application: **25.03.2026**
(21) Application number: 24202071.7
(22) Date of filing: 23.09.2024
(51) Int. Cl.: G01N 33/00, H01J 49/04

(54) **SYSTEM FOR HYDROGEN DETECTION AT LOW VOLUME MIXING RATIOS**

(71) Applicant: Advanced Monitoring Solutions AS, 0775 Oslo (NO)
(72) Inventor: Wisthaler, Armin, 0775 Oslo (NO); Mikoviny, Tomas, 0481 Oslo (NO)
(74) Representative: Schulz Junghans Patentanwälte PartGmbB

(57) **Abstract**

The invention relates to a system (1) for detecting molecular hydrogen in a gas, wherein the system (1) comprises the following components:
- A gas inlet (10) for sampling gas,
- A gas drier (2), configured to remove water from the gas
- A vacuum section (3) configured to maintain gas at a gas pressure at or below 100 mPa,
- A mass spectrometer (4) configured to determine a volume mixing ratio of molecular hydrogen in the gas comprised in the vacuum section (3),
- A first vacuum pump (5-1) configured to generate the gas pressure in the vacuum section (3),
characterized in that
the gas drier (2) is arranged between the gas inlet (10) and the vacuum section (3), wherein the gas drier (2) is configured to reduce a water mixing ratio in the gas below 3000 ppm.

## Description

The invention relates to a system for detecting molecular hydrogen in a gas.

In the art, hydrogen detection in a gas stream, e.g., for leak detection at low volume mixing ratios, is performed by mass spectrometry.

In the so-called "sniffing mode" operation, an object to be leak-tested is filled with a hydrogen-containing gas and a stream of air is sampled into the mass spectrometer from the outside of the test object.

The mass spectrometric measurement of hydrogen in a gas stream is based on the following basic principle: (1) a gas stream is sampled and brought to high-vacuum, (2) energetic electrons ionize a fraction of the gas molecules (electron ionization), (3) a mass spectrometer separates hydrogen cations, H₂⁺, with a mass to charge ratio (m/z-ratio) of two from ions with other m/z-ratios, (4) an ion detector collects H₂⁺ ions with the m/z-ratio of 2, and the thereby generated electric signal is converted into a quantitative measurement of the hydrogen volume mixing ratio in the gas stream.

State-of-the-art mass spectrometers for measuring hydrogen in a gas stream exclusively use electrons as the ionizing agents.

State-of-the-art mass spectrometers are capable of measuring hydrogen volume mixing ratios down to single-digit-ppm (ppm: parts per million) levels in a gas stream. However, mass spectrometers suffer from a significant positive bias (typically ≥1 ppm) in the measured hydrogen volume mixing ratio when the gas stream contains water at typical ambient humidity levels. This bias makes it impossible to accurately measure low volume mixing ratios of hydrogen in a humid gas and in particular in ambient air, where typical hydrogen volume mixing ratios are in the range between 0.5 and 1.5 ppm.

It is an object of the invention to overcome this problem and to provide a system for accurately measuring a hydrogen volume mixing ratio in the low-ppm and sub-ppm region. The object is achieved by the system having the features of claim 1.

Advantageous embodiments are described in the dependent claims.

According to claim 1, a system for detecting molecular hydrogen in a gas comprises the following components:
- A gas inlet for sampling gas,
- A gas drier, configured to efficiently remove water from the gas,
- A vacuum section configured to maintain gas at a gas pressure at or below 100 mPa,
- A mass spectrometer configured to detect molecular hydrogen in the gas comprised in the vacuum section,
- A first vacuum pump configured to generate the reduced gas pressure in the vacuum section, e.g. by pumping gas from the gas inlet through the vacuum section to a gas outlet, wherein
the gas drier is arranged between the gas inlet and the vacuum section, wherein the gas drier is configured to reduce a water mixing ratio in the gas below 3,000 ppm.

Throughout the specification the abbreviation "ppm" (parts per million) refers to 10⁻⁶ volume by volume.

The inventors have found that a positive measurement bias of hydrogen mixing ratios at low volume mixing ratios in a gas is caused by presence of water (H₂O) comprised by the gas. Ionization, such as electron ionization in mass spectrometry under typical conditions, partly dissociates the water molecules to form hydrogen cations, H₂⁺, which are detected by the mass spectrometer and thereby causing a positive bias in the hydrogen measurement.

The inventors have further found that in state-of-the-art mass spectrometers used for hydrogen measurements the positive bias in the hydrogen volume mixing ratios amounts to approximately 0.01% of the water vapor mixing ratio in the gas. As the Earth's atmosphere typically contains 1% to 2% (10,000-20,000 ppm) of water, any state-of the art measurement in atmospheric conditions results in a 1 ppm to 2 ppm positive bias in atmospheric hydrogen measurements.

The invention solves the problem by employing a gas drier that is arranged upstream the vacuum section and that is configured to reduce the amount of water in the gas, such that a remaining bias of residual water molecules in the gas that reach the mass spectrometer, is below a desired threshold.

In the context of the current specification the expression "upstream" particularly refers to a net flow direction of the gas once it enters the system via the gas inlet. The expression "upstream" may relate to a direction oriented against said net flow direction.

Similarly, the expression "downstream" may be understood as a direction oriented along the net flow direction.

It is understood that the notion of water comprised by the gas, particularly refers to molecular water vapor in the gas, which the invention aims to reduce.

In particular, the volume mixing ratio of water in the gas after the gas drier, is set to be below 3,000 ppm, which translates to a bias of not more than 0.3 ppm in hydrogen detection, therefore allowing determination of comparably unbiased hydrogen volume mixing ratios of gases.

Peltier cooling elements, which for example are used in the prior art for gas drying in mass spectrometry, are not capable of reducing the water mixing ratio to below 3,000 ppm. Such a gas cooler typically cools the gas to 2°C to 5°C, which corresponds to a saturation water vapor mixing ratio of 7,000 ppm to 9,000 ppm at standard atmospheric pressure (101,325 Pa). Therefore, Peltier cooler based systems in the art are not capable to achieve the goal of the invention.

Other systems in the art arrange for cryogenic cooling of the gas, e.g. by liquid nitrogen traps, in the vacuum section of such a system, which however renders the system prone to frequent maintenance such as frequent defreezing.

The system according to the invention not only allows for an accurate hydrogen volume mixing ratio measurement in atmospheric conditions but also provides a solution that has a greatly reduced need of maintenance.

According to the invention, the gas drier is arranged upstream the vacuum section, such that the water comprised by the gas, is removed from the gas before the gas enters the vacuum section.

The pressure at the gas drier may be still below atmospheric pressure, but only to a moderate degree. For example, it is conceivable that a pressure at the gas drier is in the range of atmospheric pressure down to 100Pa, or even lower. It is also conceivable, that the pressure at the gas drier is above atmospheric pressure.

The components of the system are fluidically connected, such that gas that entered the inlet of the system may be transported from one component to the next component, that may be arranged downstream.

That, is, from the inlet that gas may be guided, e.g. via a corresponding tubing, to the gas drier, where the water is removed before it directed further to the vacuum section, and from the vacuum section the gas is provided to the mass spectrometer.

A gas flow may be obtained by the vacuum section that induces a gas stream from the inlet to the vacuum section.

Further, regarding pressure values provided in the context of the current specification, it is noted that pressure values may be understood to refer to pressure at temperatures at the location, where the pressure is determined. The temperature may be a surrounding temperature of the system or a component of the system, or an ambient temperature or a temperature around or at 293K.

The vacuum section is preferably configured to maintain a so-called high vacuum. That is a pressure in the vacuum section, when the system is operating, may be in the ranges and conditions as specified in ISO 3529-1:2019.

The first vacuum pump is configured to generate the vacuum in the vacuum section. It is conceivable, that a second vacuum pump is comprised by the system, wherein said second pump is arranged downstream of the first vacuum pump, to reduce a pressure gradient between the vacuum section and the gas outlet.

According to another embodiment of the invention, the gas drier comprises a moisture exchanger with an exchange membrane.

Such membrane-based gas driers have several advantages. Membrane gas driers require comparably low maintenance, may be operated at various temperatures - in particular, it may not be required to cool the gas to very low temperatures to force condensation of the water. Further, the exchange membrane may act as an additional filter for other compounds comprised by the gas, such as ammonia, and/or hydrocarbons.

According to another embodiment of the invention, the moisture exchanger comprises an inlet and an outlet, wherein the moisture exchanger comprises a first and a second channel, that form two flow channels. The first and the second channel may be arranged concentrically with respect to each other and have a tube-like shape, so as to form for example an inner and an outer channel.

According to another embodiment of the invention, the moisture exchanger comprises a plurality of first and second channels that are arranged fluidically parallel to each other. This allows for parallel, and thus high-volume gas drying and for a more compact build space of the gas drier.

The exchange membrane forms at least a part of a wall of the first channel.

The system further is configured to flow a drying or purge gas through the second channel, in particular against a net flow direction of the gas (to be dried) flowing in the first channel. Via the exchange membrane water may be exchanged from the gas to be dried to the drying or purge gas. That is the exchange membrane is permeable for water.

This allows for an efficient and low-maintenance gas drier, that may be operated in a variety of conditions and temperatures at ambient pressure.

The exchange membrane is further configured to be impermeable for hydrogen in the gas (to be dried).

According to another embodiment of the invention the exchange membrane comprises an ionomer, such as a sulfonated tetrafluoroethylene-based fluoropolymer-copolymer (NAFION). Particularly, the exchange membrane may consist of an ionomer, such as sulfonated tetrafluoroethylene-based fluoropolymer-copolymer.

Ionomers are particularly, well-suited for exchange membranes for gas drying.

According to another embodiment of the invention, the gas drier comprises a desiccant granulate.

This embodiment may be understood as an alternative or additional embodiment to the moisture exchanger.

Desiccant granulate allows for a cost-efficient gas drying, while regenerating of the desiccant granulate may be facilitated by simply heating the granulate.

Alternatively, the desiccant granulate may be repeatedly regenerated by adjusting a pressure surrounding the granulate. This process is referred to as pressure swing absorption.

According to another embodiment of the invention, the desiccant granulate consists of one or more selected from the group:
- anhydrous calcium sulfate,
- silica gel
- activated alumina,
- calcium chloride.

This embodiment allows for highly efficient gas drying.

According to another embodiment of the invention, the gas drier comprises one or more cryogenic traps, such as liquid nitrogen traps.

According to another embodiment of the invention, the gas drier comprises two or more cryogenic traps, wherein the traps are arranged fluidically parallel.

According to another embodiment of the invention, the system comprises a switch configured to adopt at least a first and a second state, wherein in the first state, the gas is guided from the inlet to a first of the two or more cryogenic traps and particularly not to a second cryogenic trap of the two or more cryogenic traps, wherein in the second state, the gas is guided from the inlet to the second of the two or more cryogenic traps, and particularly not to the first cryogenic trap of the two or more cryogenic traps.

For example, in case the gas comprises more than 1% of water, a single cryogenic trap would quickly freeze over by the amount of water condensing in the trap, rendering the trap inoperable or inefficient. This embodiment allows alternating operation of the first or the second cryogenic trap, such that the respective other trap that is not exposed to the gas may be de-iced, e.g. by heating and purging the trap with a dry gas, such as dry air or nitrogen.

The system may comprise respective components for facilitating the de-icing.

According to another embodiment of the invention, the gas drier is configured to reduce the volume mixing ratio of water in the gas below 1,000 ppm, particularly, below 500 ppm, more particularly, below 100 ppm, even more particularly below 50 ppm or even below 10 ppm.

The lower the volume mixing ratio of water, the lower the positive bias in the measured hydrogen volume mixing ratio of the system.

Particularly, with the gas drier according to the preceding embodiments, such a drier comprising a moisture exchanger and/or a desiccant granulate, it is possible to achieve water volume mixing ratios below 100 ppm in the dried gas, which translates to a bias of the estimated hydrogen volume mixing ratio of approximately 0.01 ppm, a value that has not been reported in the art.

Accordingly, the bias of hydrogen volume mixing ratio is still only 0.1 ppm, in case the dried gas comprises 1,000 ppm of water, a value that is not attained by instruments in the art.

According to another embodiment of the invention, the dried gas at the gas drier has a temperature higher than 100K, particularly higher than 150K.

According to another embodiment of the invention, the gas drier is configured to adjust, particularly to reduce a dew temperature of the gas to a range below -30°C, particularly to range below -40°C, more particularly below-50°C.

At standard atmospheric pressure (101,325 Pa), a temperature below -30°C is equivalent to a water volume mixing ratio below 375 ppm, wherein for a temperature below -40°C the water volume mixing ratio is below 200ppm, wherein at -50°C the water volume mixing ratio is below 100 ppm.

According to another embodiment of the invention, the system comprises a hydrocarbon filter that is configured to filter hydrocarbons from the gas, particularly gaseous hydrocarbons, particularly methane, ethane, particularly wherein the hydrocarbon filter is comprised by the gas drier and/or formed as a filter unit separate from the gas drier.

This embodiment allows for removing residual sources of hydrogen bias, as hydrocarbons may falsely contribute to the count of molecular hydrogen in the gas, when analysed by the mass spectrometer due to ionization products of the hydrocarbons.

The filter may be comprised in the gas drier, e.g. for example, by selecting an appropriate exchange membrane or the filter may be embodied as a separate component that is not comprised by the gas drier. The hydrocarbon filter is preferably arranged upstream the vacuum section.

In case the hydrocarbon filter is a separate filter unit from the gas drier, that hydrocarbon filter may be arranged upstream the gas drier.

Alternatively, the filter unit, i.e. the hydrocarbon filter may be arranged downstream of the gas drier.

This embodiment benefits the filter unit, as at least some filter types may exhibit a water-sensitive performance, that is decreases as the water mixing ratio rises. Therefore, arranging the filter unit downstream provides at least for some types of filters an improved performance.

According to another embodiment of the invention, the system comprises an ammonia filter that is configured to filter ammonia from the gas, particularly wherein the ammonia filter is comprised by the gas drier and/or formed as a filter unit separate from the gas drier.

This embodiment allows for removing residual sources of hydrogen bias, as ammonia may falsely contribute to the count of molecular hydrogen in the gas, when analysed by the mass spectrometer due to ionization products of ammonia.

The filter may be comprised in the gas drier, e.g. for example, by selecting an appropriate exchange membrane or the filter may be embodied as a separate component that is not comprised by the gas drier. The ammonia filter is preferably arranged upstream the vacuum section.

In case the ammonia filter is a separate filter unit from the gas drier, that ammonia filter may be arranged upstream the gas drier.

Alternatively, the filter unit, i.e. the ammonia filter may be arranged downstream of the gas drier.

This embodiment benefits the filter unit, as at least some filter types may exhibit a water-sensitive performance, that is decreases as the water mixing ratio rises. Therefore, arranging the filter unit downstream provides at least for some types of filters an improved performance.

Ammonia particularly refers to NH₃.

According to another embodiment of the invention, the system comprises a temperature stabilizing device, that is configured to maintain a preset temperature for one or more components of the system.

The temperature stabilizing device may comprise one or more temperature sensors configured to determine a temperature, wherein the temperature stabilizing device may further comprise a heating and cooling component configured to adjust a temperature in response to the temperature determined by the one or more temperature sensors.

In addition, or alternatively, the temperature stabilizing device may comprise temperature-insulating walls forming a housing for the one or more components of the system.

The temperature stabilizing device particularly ensures that temperature-driven sensitivity or detection efficiency drifts of electronics and sensors of the components of the system are avoided.

The preset temperature may be in the range of 15°C to 25°C.

According to another embodiment of the invention, the temperature stabilizing device maintains the preset temperature within a range of ± 0.5°C, particularly ± 0.3°C, more particularly in a range of ± 0.1°C, around the preset temperature.

This embodiment allows for a more precise determination of the hydrogen volume mixing ratio, as a temperature-induced drift in an output signal of the system in particular the mass spectrometer is excluded as a source of error. The output signal is indicative of and/or comprises an information on the hydrogen volume mixing ratio. According to another embodiment of the invention, the temperature stabilizing device, comprises one or more components from a list consisting of:
- A detector of the mass spectrometer;
- Detector electronics of the mass spectrometer;
- The mass-spectrometer.

These components may therefore be kept at within the range around the preset temperature.

According to another embodiment of the invention, the system is configured to detect molecular hydrogen in the gas in volume mixing ratios from 0.01 ppm, particularly 0.05 ppm upwards.

According to another embodiment of the invention, the mass spectrometer is configured to ionize all or a fraction of the gas, to subsequently separate ionized molecular hydrogen by means of a mass-to-charge ratio separation, and to detect the corresponding mass-to-charge ratio of the ionized molecular hydrogen on a detector of the mass-spectrometer configured to generate a output signal indicative, particularly quantitative, for the hydrogen mixing ratio of molecular hydrogen in the gas. Ionization of the gas may be effected e.g. by means of electron ionization, photoionization, chemical ionization, electrical field ionization, Penning ionization. That is the invention described herein may be applied to other embodiments in which hydrogen ionization is effected via interactions with photons (photoionization), with gaseous ions (chemical ionization), with a strong electric field (field ionization), or with energetic neutrals (Penning ionization).

In particular, the mass spectrometer comprises a magnetic sector mass spectrometer, which allows for a compact and low-cost design.

According to another embodiment of the invention, the mass spectrometer comprises a quadrupole mass spectrometer, an ion trap mass spectrometer, a time-of-flight mass spectrometer or another type of m/z-separating device.

The term "m/z-separating" particularly refers to a device that separates ions by a mass-to-charge ratio (*m*/*z*).

Particularly, exemplary embodiments are described below in conjunction with the Figures. The Figures are appended to the claims and are accompanied by text explaining individual features of the shown embodiments and aspects of the present invention. Each individual feature shown in the Figures and/or mentioned in said text of the Figures may be incorporated (also in an isolated fashion) into a claim relating to the device according to the present invention.
Fig. 1 shows an exemplary embodiment of a system according to the invention;
Fig. 2 shows another exemplary embodiment of a system according to the invention; and
Fig. 3 shows exemplary embodiments of gas driers.

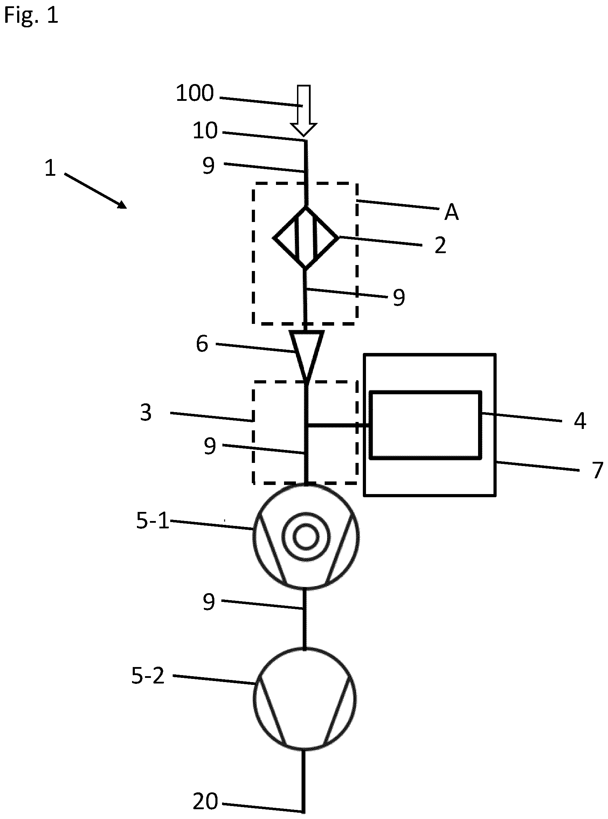

In Fig. 1 an exemplary schematic drawing of a system 1 according to the invention is depicted.

The system 1 comprises a gas inlet 10 that may be formed as an opening through which gas 100 may enter a channel system 9, e.g. a tubing, connecting the components of the system 1. Said channel system 9 is depicted as solid lines connecting the components. The system 1 is configured to determine a volume mixing ratio of molecular hydrogen in the gas entering the gas inlet 10.

The system 1 comprises a gas drier 2 arranged downstream of the gas inlet 10, wherein the gas drier 2 is connected via the channel system 9 to the gas inlet 10 and configured to operate at atmospheric or near-atmospheric pressure (indicated by box A).

The gas drier 2 is a high-efficiency gas drier 2 and configured to reduce a volume mixing ratio of water vapor in the gas below 3,000 ppm, particularly below 1,000 ppm or even lower than 500 ppm.

It is noted that conventional gas coolers, e.g. Peltier driven gas coolers, known in the art are not suitable nor configured to achieve water volume mixing ratios below 3,000 ppm.

After passing the gas drier 2, the gas comprises less than 3,000 ppm water vapor.

Following the gas drier 2 in a downstream direction of the channel system 9 there is arranged a vacuum section 3, in which a pressure of the dried gas is reduced to high-vacuum conditions. The high vacuum may be lower than 100 Pa.

For this, the system 1 may comprise a flow-restricting device 6, such as a choke, a throttle or a valve, configured to maintain a pressure gradient between the gas drier 2 and the vacuum section 3. The pressure gradient over the flow-restricting device 6 may be generated by pumping gas from the gas inlet 10 through the vacuum section 3 to a gas outlet 20 downstream of the vacuum section 3, e.g. by means of a first vacuum pump 5-1.

For this, the channel system 9 is connected to said first vacuum pump 5-1 downstream of the vacuum section 3. The first vacuum pump 5-1 allows pumping gas from the gas inlet 10 through the vacuum section 3 to a gas outlet 20. The first vacuum pump 5-1 is configured to generate and maintain the high vacuum condition in the vacuum section 3. The first vacuum pump 5-1- may be a turbomolecular pump. Downstream of the first pump 5-1 there is the gas outlet 20, through which the dried gas may exit the system 1. To reduce a pressure gradient against which the first pump 5-1 must operate, a second vacuum pump 5-2, in form of a backing pump may be arranged between the gas outlet 20 and the first vacuum pump 5-1.

For determining the hydrogen volume mixing ratio in the dried gas, a mass spectrometer 4 is comprised by the system 1, wherein the mass spectrometer 4 is connected to the high-vacuum section 3, where dried gas samples are obtained for the mass spectrometer 4.

The mass spectrometer 4 is configured to ionize the gas, e.g. by means of an electron ionization method and system. Other ionization methods may be suitable as elaborated in previous paragraphs.

To exclude systematic errors due to varying temperatures at the mass spectrometer 4, the system 1 may comprises a temperature-stabilizing device 7, that comprises the mass spectrometer 4 or parts or components thereof, such that temperature-sensitive electronics and detectors may not suffer from a temperature-induced drift.

Further, the gas drier 2 may be configured to filter hydrocarbons and/or ammonia from the gas, that due to ionization at the mass spectrometer 4 could otherwise contribute to a false hydrogen signal at the detector of the mass spectrometer 4.

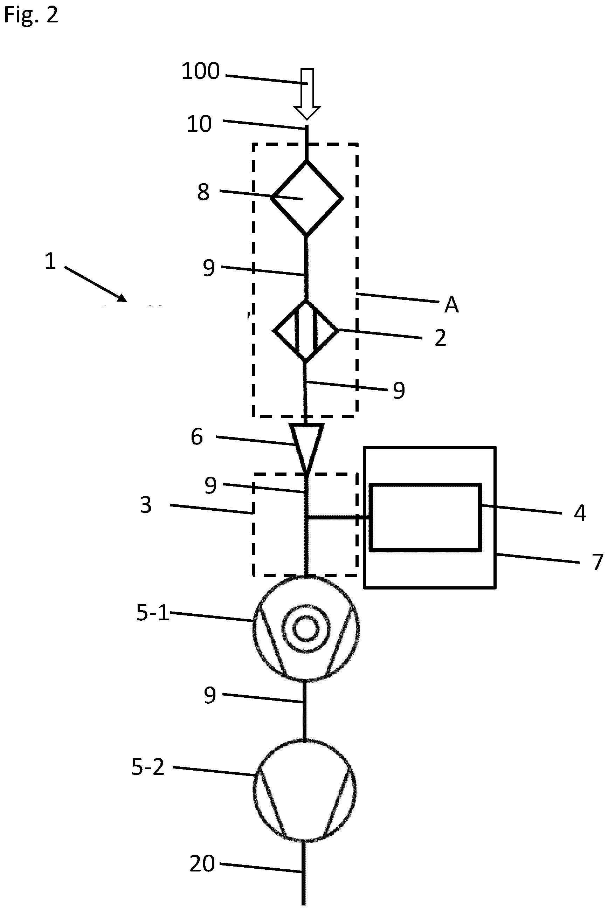

In Fig. 2 a variant of the system of Fig. 1 is shown. While the system 1 of Fig. 2 is in large parts identical to the system of Fig. 1, a difference is that upstream the gas drier 2, a separate filter unit 8 is arranged, that can operate at the same pressure conditions as the gas drier 2 and that is configured to filter hydrocarbons and/or ammonia (Alternatively, the separate filter unit 8 may be arranged downstream of the gas drier 2 (not shown)). This configuration allows for a reduced bias in hydrogen mixing ratio determination, particularly in case the gas to be tested comprises comparably large amounts of hydrocarbons or ammonia. In addition to the separate filter unit 8, the gas drier 2 may nonetheless be configured to filter for hydrocarbon and/or ammonia as well.

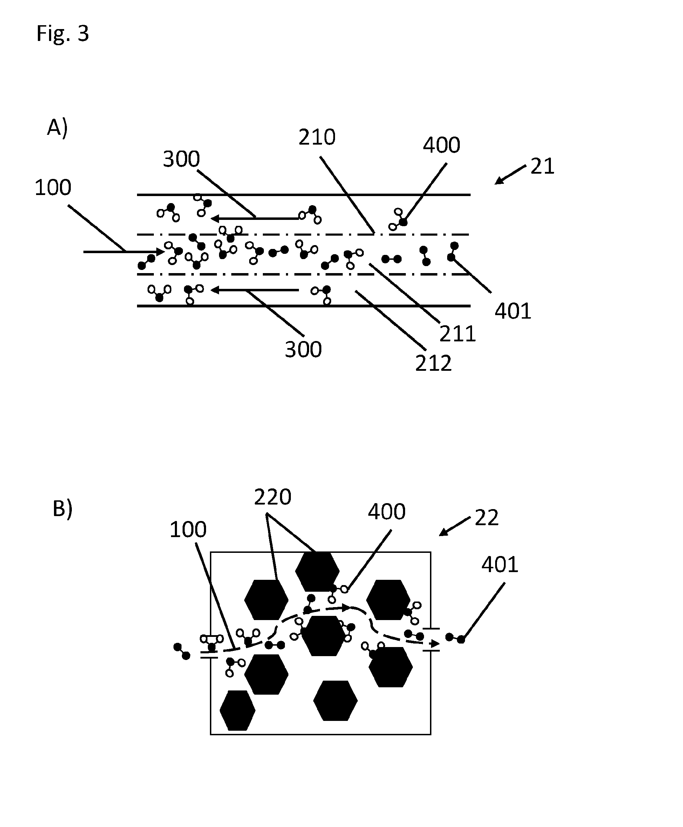

In Fig. 3 various advantageous embodiments of a gas drier 2 are depicted. In Fig. 3A, a gas drier 2 comprising a moisture filter 21 with an exchange membrane 210 is schematically depicted. In Fig. 3B a gas drier 2 comprising a desiccating granulate 220 is depicted.

In Fig. 3A the moisture filter 21 comprises two channels 211, 212, wherein a first channel 211 of the two channels extends concentrically inside a second channel 212 of the two channels. Other moisture filter/channel geometries are conceivable. The first channel 211 is configured to carry the gas 100 to be dried. The gas to be dried, comprises water 400 and molecular hydrogen 401 and flows along a gas flow direction as depicted by arrow 100. A drying or purge gas flows in the second channel 212 that encloses the first channel 211. A channel wall of the first channel 211 comprises or consists of an exchange membrane 210 permeable for water vapor 400, such that the water vapor 400 may pass from the first channel 211 to the second channel 212. At the same time, the exchange membrane is impermeable and inert with respect to molecular hydrogen 401, such that molecular hydrogen 401 is retained in the first channel 211. The drying gas, e.g. dried air, flows in the opposite direction 300 than the gas to be dried, and thus carries any water molecule 400 away, depriving the gas to be dried from water.

Advantageously, the exchange membrane 210 may be configured to further allow hydrocarbons and/or ammonia to pass to the second channel 212. The degree of water vapor reduction depends on the flow speed of the gas to be dried as well as a length of the first channel and second channel 211, 212, wherein it is possible to recirculate the gas to be dried several times through the first channel 211, if necessary. The drying gas 300 may be replenished from a dry gas source that provides drying gas with a mixing ratio of water that is lower than the mixing ratio of the gas to be dried. Said replenishing may be even facilitated with a fraction of the dried gas that may be recirculated back in the second channel 212 as the purge/drying gas (not shown).

In Fig. 3B, an embodiment of the gas drier 2 comprising a granulate drier 22 that comprises a desiccating granulate 220 is shown. The gas 100 to be dried is flown through the granulate 220, or flown by the granulate, wherein the granulate 210 is hydrophilic and deprives the gas to be dried from the water vapor 400. The granulate does not deprive the gas form molecular hydrogen 401, such that the gas once dried comprises the same hydrogen volume mixing ratio as the gas before it was dried.

When the granulate 220 is saturated, it may be replenished e.g. by heating the granulate 220 to evaporate the water. Additionally, or alternatively, replenishing the granulate's desiccant capacity may be achieved by adjusting the pressure.

Clearly, a gas drier 2 comprising both, the moisture filter 21 as well as the desiccating granulate filter 22, may yield excellent results.

It is noted that also other gas driers 2 may be suitable for reducing the volume mixing ratio of water vapor below the desired threshold of at least 3,000 ppm. For example, one or more cryogenic traps (e.g. liquid nitrogen trap) may be suitable, as long as arranged upstream and outside the vacuum section 3.

With the system 1 according to the invention, minute concentrations of molecular hydrogen in a gas may be determined accurately down to the sub ppm range.

The system 1 may be applied to measure hydrogen mixing ratios in the atmosphere or in leak detection application, where such systems operate in "sniffing mode", to detect any hydrogen escaping the system under test.

### List of Reference Signs

- 1: system
- 2: gas drier
- 21: moisture filter
- 210: exchange membrane
- 211: first channel
- 212: second channel
- 22: granulate filter
- 220: desiccant granulate
- 3: high vacuum section
- 4: mass-spectrometer
- 5-1: first pump
- 5-2: second pump
- 6: flow restrictor
- 7: temperature stabilizing device
- 8: filter unit for hydrocarbons or ammonia
- 9: channel system /tubing
- 10: gas inlet
- 20: gas outlet
- 100: gas flow
- 300: drying or purge gas flow
- 400: water
- 401: molecular hydrogen

## Claims

1. A system (1) for detecting molecular hydrogen (401) in a gas, wherein the system (1) comprises the following components:
- A gas inlet (10) for sampling gas,
- A gas drier (2), configured to remove water (400) from the gas
- A vacuum section (3) configured to maintain gas at a gas pressure at or below 100 mPa,
- A mass spectrometer (4) configured to determine a volume mixing ratio of molecular hydrogen (401) in the gas comprised in the vacuum section (3),
- A first vacuum pump (5-1) configured to generate the gas pressure in the vacuum section (3),
**characterized in that**
the gas drier (2) is arranged between the gas inlet (10) and the vacuum section (3), wherein the gas drier (2) is configured to reduce a water mixing ratio in the gas below 3,000 ppm.

2. The system (1) according to claim 1, the gas drier (2) comprises a moisture exchanger (21) with an exchange membrane (210).

3. The system (1) according to claim 2, wherein the exchange membrane (210) comprises a ionomer.

4. The system (1) according to one of the preceding claims, wherein the gas drier (2) comprises a desiccant granulate (220).

5. The system (1) according to one of the preceding claims, wherein the desiccant granulate consists of one or more selected from the group:
- anhydrous calcium sulfate,
- silica gel
- activated alumina
- -calcium chloride.

6. The system (1) according to one of the preceding claims, wherein the gas drier (2) is configured to reduce the volume mixing ratio of water in the gas below 1,000 ppm.

7. The system (1) according to one of the preceding claims, wherein the gas drier (2) is configured to adjust a dew temperature of the gas to a range below - 30°C.

8. The system (1) according to one of the preceding claims, wherein the system (1) comprises a hydrocarbon filter (8) that is configured to filter hydrocarbons from the gas.

9. The system (1) according to one of the preceding claims, wherein the system (1) comprises an ammonia filter (8) that is configured to filter ammonia from the gas.

10. The system (1) according to one of the preceding claims, wherein the system (1) comprises a temperature stabilizing device (7), that is configured to maintain a preset temperature for one or more components of the system (1).

11. The system (1) according to claim 10, wherein the temperature stabilizing device (7) is configured to maintain the preset temperature within a range of ± 0.5°C around the preset temperature.

12. The system (1) according to claim 10 or 11, wherein the temperature stabilizing device (7) comprises one or more components from a list consisting of:
- a detector of the mass spectrometer (4);
- detector electronics of the mass spectrometer (4);
- the mass-spectrometer (4).

13. The system (1) according to one of the preceding claims, wherein the system (1) is configured to detect molecular hydrogen (401) in the gas in volume mixing ratios from 0.01 ppm.

14. The system (1) according to one of the preceding claims, wherein the mass spectrometer (4) is configured to ionize the gas, to subsequently separate ionized molecular hydrogen by means of a mass-to-charge ratio separation, and to detect the corresponding mass-to-charge ratio of the ionized molecular hydrogen on a detector of the mass-spectrometer (4) configured to generate a output signal indicative for the hydrogen volume mixing ratio of molecular hydrogen in the gas.
